# EUROPEAN PATENT SPECIFICATION

(11) **EP 1 639 372 B1**
(45) Date of publication and mention of the grant of the patent: **02.12.2009**
(21) Application number: 04764229.3
(22) Date of filing: 02.07.2004
(51) Int. Cl.: G01N 33/569

(54) **DIAGNOSTIC TEST**
DIAGNOSTISCHER TEST
TEST DE DIAGNOSTIC

(30) Priority: 02.07.2003 GB 0315512
(43) Date of publication of application: 29.03.2006
(73) Proprietor: NEUTRACT, 75014 Paris (FR)
(72) Inventor: BENVENISTE, Jacques, F-75014 Paris (FR)
(74) Representative: Enderlin, Eric André
(86) International application number: PCT/EP2004/009242
(87) International publication number: WO 2005/005987

(56) References cited:
- WO-A-00/52472
- WO-A-96/40869
- ABDELILAH S ET AL: "Functional expression of IL-9 receptor by human neutrophils from asthmatic donors: role in IL-8 release." JOURNAL OF IMMUNOLOGY (BALTIMORE, MD. : 1950) 15 FEB 2001, vol. 166, no. 4, 15 February 2001 (2001-02-15), pages 2768-2774, XP002298341 ISSN: 0022-1767

## Description

This invention relates to a cellular diagnostic test for the detection of infection and inflammation.

It is difficult to name a disease where inflammation is not present. The main actor in the creation and perpetuation of inflammation is the blood white cell (leucocyte) named polymorphonuclear neutrophil (PMN). PMNs are by far the most numerous of blood leucocytes. They also play a key role in the defence against microbes, as shown by the onset of deadly infections when they are impaired or too low in number. The paradox is that for this pivotal cell there exists no biological test that is both recognised by academic medicine and routinely accessible to the patients.

Consequently there are many gaps when it comes to understand the pathogenesis of many diseases, in turn resulting in the lack of proper diagnostic procedures. A good example can be found in a syndrome named "food intolerance" or "food sensitivity" that describes any clinically abnormal response attributed to an exposure to a food or food component, whether immunological or non-immunological.

Food intolerance reflecting PMN activation may be an underlying mechanism contributing to some serious chronic and even autoimmune diseases. Furthermore, several studies have shown that many conditions such as migraine, irritable bowel syndrome, rheumatoid arthritis, respond to dietary modification. However, the offending foods are not identifiable by usual immunological methods.

Several methods of assaying leucocyte activation in whole blood are offered to selected groups of patients, generally restricted to food intolerance:
1) the antigen leucocyte cellular antibody test (ALCAT), which is intended for the determination of the effects of multipathogenic mechanisms involved in food sensitivities. This method suffers from the disadvantage that it requires costly and specialised equipment and relatively large samples of whole blood, e.g. between 15 and 20 mL.
2) The Yorke Test is confined to measuring a class of antibodies, the IgG, based on the debatable assumption that food intolerance is exclusively a reaction due to the immune system.
3) Other methods measure the release of chemical mediators. One such example is the Food Allergen Cellular Test (FACT), where food sensitised leucocytes release an inflammatory group of molecules, the leukotrienes, which it is claimed reflect allergic responses but are unable to distinguish between food allergy and intolerance.

Document WO/94869 discloses a method for screening infectious diseases that involves visualizing the surface binding of an infectious agent to naturally-occurring polymorphonuclear leukocytes to detect the infection.

Document from Abdelilah S et al :" Functional expression of IL-9 receptor by human neutrophils from asthmatic donors): role in IL-8 release." (Journal of immunology, 15 FEB 2001, vol. 166, no. 4., pages 2768-3774) discloses the role of PMNs in human pathogenesis of allergic disease.

Document WO 00/52472 discloses a rapid assay for infection in small children.

Practically all of the prior art methods of diagnosing PMN activation, e.g. in food intolerance are indirect methods which do not address directly the activation of the cell.

By contrast, we have invented and developed a new diagnostic method, which overcomes or substantially mitigates the disadvantages of prior art methods, whilst at the same time giving reliable measures of the state of PMN. It then becomes possible to explore infection, inflammation and other syndromes such as food intolerance by directly measuring PMN activation.

According to the invention we provide a method of diagnosing the level of PMN activation following incubation of a leucocyte sample prepared from whole blood. PMN activation may be either spontaneous (thus reflecting the basic state of the cell in circulating blood) or after exposure to a classical PMN activating agent such as phorbol-myristate acetate (PMA) or to substances suspected of acting on PMNs such as food extracts.

The method allows detecting whether or not PMNs in the sample have become activated following incubation in the presence of the said compounds.

The invention relates to a method of diagnosing intolerance by a subject to a specified substance which comprises incubating a leucocyte sample prepared from whole blood drawn from the subject with an extract of said substance and detecting whether or not PMNs in the sample have become activated.

A number of methods can be used to determine if PMNs have become activated.

The change in PMN morphology which results from activation can be determined optically, e.g. using a microscope.

According to an embodiment, the PMN activation is detected optically.

However, although this method is accurate, it is very time consuming, which makes it unfit for routine testing. Alternative methods of detecting PMN activation include luminescence, detecting the uptake of dyes or fluorescent markers, photometry, detecting the release of cytokines or other bioactive molecules (e.g. leukotrienes), microbial proteins (e.g. defensins) and/or free radicals (e.g. reactive oxygen metabolites).

According to an embodiment, PMN activation is detected by determining whether they adhere to surface. This makes use of the fact that PMNs tend to become more adhesive on activation. As such, a preferred method of detecting PMN activation is to monitor the increased adhesiveness of activated PMNs, relative to inactivated ones. In particular, activated PMNs tend to adhere to plastic surfaces, e.g. the surface of a well in a 96-well titre plate or in a sample tube, for example an Eppendorf^{®} tube. Thus, PMN adhesion may be determined by assaying adhesion to a plastic surface that may be a plastic multi-well titre plate that may contains 96 wells. Accordingly, the number of PMNs adhering to a plastic surface can be used as a measure of PMN activation.

Adhesion may also be detected by lysis and assaying for one or more intracellular markers for PMNs. The one or more markers may be selected from acid hydrolases, myeloperoxidases, lysozyme, lactoferrin, neutral proteases, serine proteases and lactic dehydrogenase.

According to an embodiment, the plastic surface is washed to remove unreacted cells, after incubation of the sample with the food extract but before assaying adhesion.

In fact, in general, when the PMN adhesiveness is determined by detecting adhesion to any plastic surface, such as the well of 96-well plate, non-adhering cells are removed by vigorous washing. The number of PMNs adhering to the plastic surface can be assayed by several methods, e.g. those methods mentioned above.

When the surface is a well, e.g. in a 96-well titre plate, the well can be scored turbidometrically. However, preferably, the number of cells remaining after adhesion and washing is detected by lysis and assaying for one or more intracellular PMN components.

Methods of cell lysis are well known in the art and include adding a detergent, e.g. Triton X100, to adhered cells, thereby releasing intracellular components into the cell suspending medium. Several intracellular PMN markers for activation include acid hydrolases, myeloperoxidases, lysozyme, lactoferrin, neutral and serine proteases. A preferred marker is the cytoplasmic enzyme lactic dehydrogenase (LDH), as the ratio of extracellular to intracellular enzyme is high, of the order 1:500.

The method is preferably carried out using a 96 well titre plate, allowing an array of food extracts to be investigated in run, together with suitable controls.

### Example

The following example is about detection of neutrophil reactivity to foodstuffs. All substances capable of activating neutrophils e.g. PMA, (always used as a positive control, see below), anaphylatoxins, lectins, hetero- or auto-antigens... can be used in the same manner as food stuffs to measure their effect on neutrophil adherence.

### 1. Preparing semi-purified leucocytes

A sample of whole blood (2 to 10 ml) is drawn from subjects being tested by phlebotomy into plastic tubes containing a Ca²⁺-chelating anticoagulant e.g. CPDA or ACD. An aliquot of 270µL Plasmagel® is added followed by gently mixing. Tubes are tilted at an angle of 45° and left for 1 hour in this position. The erythrocytes (red blood cells) sediment during this time leaving the white blood cell fraction in the plasma/plasmagel layer which now constitutes the supernatant.

Cells in supernatant are counted using a microscope: a 10µL aliquot of supernatant is mixed gently by inversion with 490µL of toluidine blue solution in a small plastic tube. A haemocytometer chamber is prepared, the dyed cells are introduced and the liquid volume fixed by placement of a cover slip. Cells are identified into respective types and counted.

Cells are then diluted with physiological saline in order to obtain an average of 40,000 cells. This number may vary between ranges of 15,000-80,000 which are suitable to provide adequate responses in subsequent assays.

### 2. Preparing food extracts and PMA dilutions

Freeze dried raw, whole foodstuffs are available commercially and are stored at -20°C before use. To be used in the test, they are submitted to water extraction for several hours, centrifuged and filtered. The resulting filtrate constitutes the stock solution and can be used for up to one month when stored at 2-8°C. Appropriate aliquots of stock are then diluted with 0.9 % w/v saline to produce working solutions ready for use in the diagnostic test. These solutions are stable at 2-8°C for two days.

Other extraction procedures can be used depending to some extent on the protocols followed by the person or laboratory carrying out the test.

PMA (positive control) is prepared by adding 150 µl dimethylsulfoxide (DMSO, obtained from Sigma® to 10 µl PMA (same source) frozen stock solution (1.6 mM) in order to obtain a 10⁻⁴ M solution. Serial 10-fold dilutions of 100 µl PMA from 10⁻⁴ to 10⁻¹⁰ M are prepared in DMSO. Agitate on vortex for no more than 5 sec between dilutions.

### 3. Reacting the cells with PMA and a library of food extracts

100µL aliquots of a library of 81 different food extracts are dispensed into a standard 96-well microtitre plate, (polystyrene) from working dilutions prepared fresh each day from stock solutions previously prepared according to conventional methods. Twelve blanks (consisting in the suspending solution e.g. saline or saline/DMSO) are also dispensed in the plate as controls. Each of the 3 remaining wells out of the 96 available in a plate receive 1 dilution of PMA in descending order, to be chosen from 10⁻¹⁰ to 10⁻⁶ M.

Aliquots (100µL) of cell supernatant are then added to each well followed by gentle mixing for 1 min. Plates are then centrifuged at 700 rpm, for 4 min, then incubated for 1 hour at 37°C.

Each plate is then inverted over a sink, tapped vigorously and blotted onto tissue paper, then washed twice with saline in order to remove unbound cells. Aliquots of 100µL of 1% Triton X100 detergent are then added to each well and mixed for 2 min.

### 4. Assay for adherent PMNs.

Since the level of LDH in TritonX-treated cell suspension is proportional to the number of cells adherent to plastic wall, the number of adherent PMNs is measured by assaying for LDH using conventional methods known per se. For example, LDH developing reagent (100µL) is added to each well followed by 30 min incubation, with the plates kept in darkness. The developed colour is then read in a plate reader and absorbances are recorded for a bichromatic read at 492 and 620nm. A suitable LDH reagent is that obtainable from Roche as the 'Cytotoxicity Detection Kit' Cat No 1 644 793.

Results are obtained by the following procedure: absorbance differences are calculated for each food extract between spectrophotometric reads at 492nm subtracted from the reference read at 620nm. This is termed the response. The same is repeated for the control population, (n=12), to determine a baseline response from which a significance level at 2.5 standard deviation (SD) is calculated. Reactive foods or other substances are identified as those that give a statistically significant response above the baseline response plus 2.5 SD.

Outlier rejection in the controls is set at 100mAU (absorbance units) and if more than 3 outliers occur then the run is rejected. A list of food extracts or other substances is compiled in ranked descending order of the absorbance response. Foods observed to be above the level of significance are identified as those causing food intolerance. These are listed in descending order of magnitude and constitute the result. Non-reactive substances are listed separately.

### Interpretation

The basal state of neutrophil reactivity is indicated by the results obtained on the 12 control wells which reflect the in vivo state of the cells. Any ongoing inflammation (whatever the cause, reaction to foods, acute or chronic disease) or infection will result in an elevated basal reactivity.

The reaction to PMA indicates the cells are reactive, thus constituting an index of the quality of the blood sample and subsequent manipulations. Its level must be compared to the mean reactivity of normal neutrophils in order to detect overreactivity of the cell population upon PMA stimulation.

Reactivity to foodstuffs are analysed as described above.

## Claims

1. A method of diagnosing intolerance by a subject to a specified substance which comprises incubating a leucocyte sample prepared from whole blood drawn from the subject with an extract of said substance and detecting whether or not polymorphonuclear leukocytes (PMNs) in the sample have become activated, wherein the step of detecting comprises determining whether or not PMNs in the sample have become adhesive, the method being **characterized in that** PMN adhesion is determined by assaying adhesion to a plastic surface.

2. A method according to Claim 1, wherein the plastic surface is a plastic multi-well titre plate.

3. A method according to Claim 2, wherein the titre plate contains 96 wells.

4. A method according to any of the claims 1 through 3, wherein the plastic surface is washed to remove unreacted cells, after incubation of the sample with the food extract but before assaying adhesion.

5. A method according to Claim 4, wherein adhesion is detected by lysis and assaying for one or more intracellular markers for PMNs.

6. A method according to Claim 5, wherein the one or more markers are selected from acid hydrolases, myeloperoxidases, lysozyme, lactoferrin, neutral proteases and serine proteases and lactic dehydrogenase.

7. A method according to any of claims 1 through 6, wherein PMN activation is detected optically.

8. A method according to any of claims 1 through 7, wherein the PMN activation is detected by luminescence, detecting the uptake of dyes or fluorescent markers, photometry, detecting release of cytokines or by detecting release of bioactive molecules, microbial proteins and/or free radicals.

## Patentansprüche

1. Methode zur Diagnostizierung der Unverträglichkeit einer Person gegenüber einer speziellen Substanz, die die Inkubation einer Leukozytenprobe mit einem Extrakt der besagten Substanz enthält, die aus dem Gesamtblut vorbereitet wurde, das der Person entnommen wurde, um festzustellen, ob polymorphkernige Leukozyten (PMN) in der Probe aktiviert worden sind oder nicht; der Erfassungsschritt besteht dabei unter anderem darin, festzulegen, ob die PMN in der Probe anhaftend geworden sind oder nicht, wobei das Verfahren **dadurch gekennzeichnet ist, dass** die Anhaftung der PMN **dadurch** bestimmt wird, dass man die Anziehung auf einer Kunststoffoberfläche untersucht.

2. Methode nach Anspruch 1, bei der die Kunststoffoberfläche eine Mikrotiterplatte mit mehreren Näpfchen aus Kunststoff ist.

3. Methode nach Anspruch 2, bei der die Mikrotiterplatte 96 Näpfchen enthält.

4. Methode nach irgendeinem der Ansprüche 1 bis 3, bei der die Kunststoffoberfläche gewaschen wird, um Zellen, die nicht reagiert haben, nach der Inkubation der Probe mit dem Lebensmittelextrakt, jedoch vor dem Anhaftungsversuch zu entfernen.

5. Methode nach Anspruch 4, bei der die Anhaftung durch eine Lysis und eine Untersuchung eines oder mehrerer intrazellulärer Merker der PMN erfasst wird.

6. Methode nach Anspruch 5, bei der ein oder mehrere Merker aus sauren Hydrolasen, Myeloperoxydasen, Lysozym, Lactoferrin, neutralen Proteasen und serinen Proteasen und Lactatdehydrogenase ausgewählt werden.

7. Methode nach irgendeinem der Ansprüche 1 bis 6, bei der die Aktivierung der PMN optisch erfasst wird.

8. Methode nach irgendeinem der Ansprüche 1 bis 7, bei der die Aktivierung der PMN durch Lumineszens erfasst wird, durch die Absorbtion der Einfärbungen oder der fluoreszenten Merker, durch Lichtstärkemessung durch Erfassung der Freisetzung von Zytokinen, oder durch die Erfassung der Freisetzung von bioaktiven Molekülen, von mikrobischen Proteinen und/oder freien Radikalen.

## Revendications

1. Procédé de diagnostic d'une intolérance d'un sujet à une substance précise qui comprend incuber un échantillon de leucocytes préparé à partir de sang entier prélevé chez le sujet avec un extrait de ladite substance et détecter si des leucocytes polymorphonucléaires (PMN) dans l'échantillon sont devenus activés ou non, dans lequel l'étape de détection comprend déterminer si les PMN dans l'échantillon sont devenus adhésifs ou non, le procédé étant **caractérisé en ce que** l'adhésion des PMN est déterminée en testant l'adhésion à une surface plastique.

2. Procédé selon la revendication 1, dans lequel la surface plastique est une plaque de titrage multipuits en plastique.

3. Procédé selon la revendication 2, dans lequel la plaque de titrage contient 96 puits.

4. Procédé selon l'une quelconque des revendications 1 à 3, dans lequel la surface plastique est lavée pour retirer les cellules n'ayant pas réagi, après l'incubation de l'échantillon avec l'extrait alimentaire, mais avant l'essai d'adhésion.

5. Procédé selon la revendication 4, dans lequel l'adhésion est détectée par une lyse et un dosage d'un ou de plusieurs marqueur(s) intracellulaire(s) des PMN.

6. Procédé selon la revendication 5, dans lequel le ou les marqueur(s) est(sont) choisi(s) parmi les hydrolases acides, les myéloperoxydases, un lysozyme, la lactoferrine, les protéases neutres et les sérine protéases et la lactate-déshydrogénase.

7. Procédé selon l'une quelconque des revendications 1 à 6, dans lequel l'activation des PMN est détectée optiquement.

8. Procédé selon l'une quelconque des revendications 1 à 7, dans lequel l'activation des PMN est détectée par luminescence, détection de l'absorption de colorants ou de marqueurs fluorescents, photométrie, détection de la libération de cytokines ou par détection de la libération de molécules bioactives, de protéines microbiennes et/ou de radicaux libres.
